(19) Europäisches Patentamt
European Patent Office
Office européen des brevets

(11) EP 0 839 513 B1

(12) **EUROPÄISCHE PATENTSCHRIFT**

(45) Veröffentlichungstag und Bekanntmachung des Hinweises auf die Patenterteilung:
**08.09.1999 Patentblatt 1999/36**

(51) Int. Cl.$^6$: **A61K 7/06**

(21) Anmeldenummer: **97110978.0**

(22) Anmeldetag: **02.07.1997**

(54) **Haarreinigungsmittel mit Festigungseigenschaften**

Hair cleaning composition with fixing properties

Produit pour le nettoyage des cheveux à pouvoir fixant

(84) Benannte Vertragsstaaten:
**DE ES FR GB IT**

(30) Priorität: **16.10.1996 DE 19642623**

(43) Veröffentlichungstag der Anmeldung:
**06.05.1998 Patentblatt 1998/19**

(73) Patentinhaber:
**Wella Aktiengesellschaft**
**64274 Darmstadt (DE)**

(72) Erfinder:
- **Karlen, Thomas, Dr.**
**3013 Bern (CH)**
- **Schmenger, Jürgen**
**64331 Weiterstadt (DE)**
- **Steinbrecht, Karin, Dr.**
**64372 Ober-Ramstadt (DE)**

(56) Entgegenhaltungen:
**WO-A-93/23009**
**WO-A-95/03776**
**WO-A-97/12587**
**WO-A-97/12593**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7/3.6

## Beschreibung

Gegenstand der vorliegenden Erfindung ist ein Haarreinigungsmittel mit einem Gehalt an mindestens einem nichtionischen, wasserunlöslichen Vinyl/Silikon Copolymer mit einem Grundgerüst aus polymeren Siloxaneinheiten und Seitenketten aus polymeren Vinyleinheiten sowie mit einem Gehalt an mindestens einem waschaktiven Tensid.

Haarreinigungsmittel enthalten üblicherweise neben den für die Reinigungswirkung ursächlichen Tensiden haarpflegende Zusätze, die gewährleisten sollen, daß das Haar sowohl im nassen als auch im trockenen Zustand gut kämmbar ist, eine verminderte statische Aufladung zeigt und einen weichen natürlichen Griff besitzt. Während die waschwirksamen amphoteren oder anionischen Tenside eines Shampoos eine gute Reinigungswirkung der Haare erlauben, ist es jedoch generell schwierig, der Frisur nach dem Waschen eine ansprechende dauerhafte Form zu verleihen, ohne daß weitere Mittel verwendet werden. Um nach der Haarwäsche neben der Reinigung eine gute Frisierbarkeit des Haares und einen möglichst dauerhaften Sitz der Frisur zu gewährleisten, werden nach der Haarwäsche mit einem Shampoo deshalb üblicherweise Haarfestiger in Form einer wäßrig-alkoholischen Polymerlösung, eines Gels, eines Schaums oder eines Haarsprays aufgebracht, die nicht wieder ausgespült werden. Dieses Verfahren ist für den Anwender zeit- und kostenintensiv, da er zwei Mittel, ein Haarreinigungsmittel und einen Haarfestiger benötigt und diese in zwei Arbeitsgängen anwenden muß.

Es sind Haarreinigungsmittel bekannt, die wasserlösliche kationische, anionische, amphotere oder nichtionische Polymere enthalten. Mit diesen Mitteln wird jedoch lediglich eine sehr schwache Festigung erreicht, da die Ladungen im Polymer ein Ausspülen der Festigersubstanz erleichtern. Die wasserlöslichen Polymere in diesen Mitteln haben deshalb im wesentlichen eine pflegende Wirkung.

Durch Festbestandteile, die in den Haarbehandlungsmitteln nicht löslich sind, wurde versucht, die Festigungsleistung zu steigern. Der Nachteil dieser Mittel liegt in einer Belastung des Haares durch Aufbau von Rückständen, welche sich bei einer Mehrfachanwendung ergeben.

Ein anderer Weg ist durch den Einsatz von Acrylat/Silikon-Copolymeren gegeben. Bekannt sind hydrophile Vinylpolymer-Grundgerüste, die hydrophobe Silikon-Seitenketten tragen. Die zu erzielende Festigung mit diesem Polymertyp ist allerdings auch schwach, weil sie entweder anionische ethylenisch ungesättigte Monomere (z. B. Acrylsäure) oder hydrophile ethylenisch ungesättigte Monomere (z. B. Dimethylaminoethyl methacrylat) enthalten (EP-OS 412 704, EP-OS 412 707). Dies erlaubt zwar, diese Polymere in Shampooformulierungen einzubringen, erhöht aber gleichzeitig die Löslichkeit des Polymers in Wasser und somit seine Ausspülbarkeit. In der WO 93/23009 und der WO 95/03776 wurde der Einsatz von Acrylat/Silikon-Copolymeren, welche hydrophile Vinylpolymer-Grundgerüste und Silikon-Seitenketten tragen, zur Erzielung von pflegenden Eigenschaften in Shampoos beschrieben. Festigende Eigenschaften dieser Polymere in Shampooformulierungen wurden weder in WO 93/23009 noch in WO 95/03776 offenbart.

Die WO 93/23009 und die WO 95/03776 beschreiben unter anderem die Herstellung von Polymeren, die im Gegensatz zu allen oben beschriebenen Polymertypen ein Silikon-Grundgerüst (silicon-backbone) mit Acrylatseitenketten aufweisen. Durch ihr Silikon-Grundgerüst vermitteln Filme dieser Polymere vor allem pflegende Eigenschaften. Es wurde der Einsatz dieser nichtionischen, hydrophoben Polymere zur Erzielung von pflegenden Wirkungen in weitgehend wasserfreien Kosmetikformulierungen wie Lippenstiften, Lidschattenstiften und Antitranspirantsprays oder in W/O Emulsionen, wobei das hydrophobe Polymer in der Ölphase gelöst ist, beschrieben. Ein Einsatz dieser Polymere in Shampooformulierungen oder eine festigende Wirkung in Kombination mit Tensiden wurde nicht beschrieben.

Es bestand daher die Aufgabe, ein Haarreinigungsmittel zur Verfügung zu stellen, welches neben einer haarreinigenden Wirkung gleichzeitig eine ausgeprägte haarfestigende Wirkung aufweist, wobei das Haar auch bei Mehrfachanwendungen nicht durch den Aufbau von Rückständen übermäßig belastet wird.

Es wurde nun gefunden, daß die gestellte Aufgabe gelöst wird durch ein Haarreinigungsmittel mit einem Gehalt an

(a) mindestens einem nichtionischen, wasserunlöslichen Vinyl/Silikon Copolymer mit einem Grundgerüst aus polymeren Siloxaneinheiten und Seitenketten aus polymeren Vinyleinheiten und

(b) mindestens einem waschaktivem Tensid, ausgewählt aus der Gruppe, bestehend aus nichtionischen, anionischen, kationischen und amphoteren Tensiden.

Das Vinyl/Silikon Copolymer ist in dem erfindungsgemäßen Mittel vorzugsweise in einer Menge von 0,1 bis 30 Gewichtsprozent und das Tensid vorzugsweise in einer Menge von 3 bis 50 Gewichtsprozent enthalten.

Geeignete Vinyl/Silikon Copolymere sind beispielsweise solche der allgemeinen Formel (I)

$$R^1R^2R^3Si\text{-}[O\text{-}SiR^4R^5\text{-}]_nO\text{-}SiR^6R^7R^8 \qquad (I),$$

wobei die Reste $R^1$ bis $R^8$ gleich oder verschieden sein können und unabhängig voneinander für eine Alkyl-, Aryl-,

Alkylaryl-, Alkoxy-, Alkylamino-, Fluoroalkyl-, Wasserstoff-, Hydroxy- oder für eine ZSA-Gruppe stehen, Z für eine divalente Verbindungsgruppe, S für Schwefel und A für ein aus nichtionischen Monomeren aufgebautes Vinylpolymer-Segment steht und n eine Zahl größer oder gleich 5 bedeutet mit der Maßgabe, daß mindestens einer der Reste $R^1$ bis $R^8$ für eine ZSA-Gruppe steht.

Geeignete nichtionische Monomere des Vinylpolymer-Segments A sind vorzugsweise ausgewählt aus niedrigpolaren Estern der Acrylsäure oder der Methacrylsäure mit Alkoholen kurzer bis mittlerer Kettenlänge, die das Polymer im wesentlichen wasserunlöslich machen. Geeignete Alkohole sind beispielsweise Ethanol, 1-Propanol, 2-Propanol, 1-Butanol, 1,1-Dimethylethanol, 2-Methyl-1-propanol, 1-Pentanol, 2-Pentanol, 3-Pentanol, 2-Methyl-1-butanol, 3-Methyl-1-butanol, 2-Hexanol, 3-Methyl-1-pentanol, 3-Methyl-1-pentanol, Cyclohexanol, 2-Ethyl-1-butanol, 3-Heptanol, Benzylalkohol, 2-Octanol, 6-Methyl-1-heptanol, 2-Ethyl-1-hexanol, 3,5-Dimethyl-1-hexanol, 3,5,5-Trimethyl-1-hexanol, 1-Decanol, 1-Dodecanol und Ähnliche. Vorzugsweise enthalten die Alkohole 1 bis 18 Kohlenstoffatome, besonders bevorzugt sind Alkohole mit 1 bis 12 Kohlenstoffatomen.

Kleine Mengen an copolymerisierbaren Monomeren wie Styrol, Vinylester, Vinylchlorid, Vinylidenchlorid, Acrylonitril, Methacrylonitril, Acryloxypropyltrimethoxysilan, Methacryloxypropyltrimethoxysilan, andere Acryloyl-Monomere und strukturell ähnliche Monomere können verwendet werden.

Beispiele besonders bevorzugter Monomere beinhalten Isooctylmethacrylat, Isononylmethacrylat, 2-Ethylhexylmethacrylat, Laurylmethacrylat, i-Pentylmethacrylat, n-Butylmethacrylat, i-Butylmethacrylat, Methylmethacrylat, Ethylmethacrylat, t-Butylmethacrylat, Tridecylmethacrylat, Stearylmethacrylat und strukturell ähnliche Monomere sowie Gemische der oben definierten Monomere.

Hochpolare, nichtionogene Monomere können zu einem kleinen Teil im Vinylpolymer-Segment A copolymerisiert sein, wenn dabei das gemäß Anspruch (A) definierte Polymer im wesentlichen wasserunlöslich bleibt. Beispiele hochpolarer, nichtionogener Monomere beinhalten nichtionogene Acrylate oder Methacrylate mit mindestens einer hochpolarisierbaren Gruppe wie einer Hydroxyl-, einer Alkoxy-, einer Aminogruppe (primär, sekundär oder tertiär) oder eines Alkenyl-Heterozyklus Vinylpyrrolidon oder Monomere mit Polyethylenoxid- oder Polypropylenoxidseitenketten. Das Vinyl/Silikon Copolymer ist jedoch vorzugsweise frei von Gruppen, welche durch Copolymerisation von hochpolaren, nichtionogenen Monomeren in das Vinylpolymer-Segment A herrühren.

Das Vinylpolymer-Segment A des Vinyl/Silikon Copolymers ist vorzgsweise frei von anionischen, kationischen oder amphoteren Gruppen, welche durch Einpolymerisieren von anionischen, kationischen oder amphoteren Monomeren herrühren.

Die Herstellung von erfindungsgemäß einsetzbaren Vinyl/Silikon Copolymeren ist in der WO 93/23009 und in der WO 95/03776 beschrieben.

Besonders bevorzugt als Polymere für das erfindungsgemäße Haarbehandlungsmittel sind Dimethylsiloxan/Methyl-3-mercaptopropyl-siloxan/Isobutylmethacrylat-Copolymere (CTFA-Bezeichnung: Polysilicone-6).

Ein geeignetes Produkt wird beispielsweise unter der Bezeichnung Silicone „Plus" Polymer VS 70 von der Firma 3M, St. Paul, Minnesota/USA vertrieben.

Das anionische Tensid der Komponente (b) ist bevorzugt ausgewählt aus den Alkali- oder Erdalkalisalzen der $C_{10}$- bis $C_{18}$-Alkylsulfate, der $C_{10}$- bis $C_{18}$-Alkylsulfonate, der $C_{10}$- bis $C_{18}$-Alkylbenzolsulfonate, der $C_{10}$- bis $C_{18}$-Xylolsulfonate und der mit 1 bis 10 Ethylenoxideinheiten ethoxylierten $C_{10}$- bis $C_{18}$-Alkylethersulfate, den ethoxylierten Sulfobernsteinsäurehalbestern der allgemeinen Formel (II)

$$R^1(OCH_2CH_2)_m\text{-}O_2C\text{-}CH_2\text{-}\underset{\displaystyle SO_3M}{\underset{|}{CH}}\text{-}COOM \qquad (II),$$

wobei $R^1$ einen $C_{10}$- bis $C_{18}$-Alkylrest bedeutet, M ein Alkali- oder Erdalkalikation darstellt und m eine ganze Zahl von 1 bis 10 bedeutet und den Alkylethercarboxylaten der allgemeinen Formel (III)

$$R_2(OCH_2CH_2)_n\text{-}OCH_2COOM \qquad (III),$$

wobei $R_2$ einen $C_{10}$ bis $C_{18}$-Alkylrest bedeutet, M ein Alkali- oder Erdalkalikation darstellt und n eine ganze Zahl von 1 bis 20 bedeutet, wobei die Alkali- und Erdalkalisalze der mit 1 bis 10 Ethylenoxideinheiten ethoxylierten $C_{10}$- bis $C_{18}$-Alkylethersulfate besonders bevorzugt sind.

Von den als Komponente (b) des erfindungsgemäßen Mittels geeigneten Alkylsulfaten ist Natriumlaurylsulfat, das beispielsweise von der Firma Henkel KGaA, Düsseldorf/Deutschland unter der Handelsbezeichnung Texapon® K12 vertrieben wird, bevorzugt. Von den geeigneten Alkylsulfonaten ist das Gemisch der Natriumsalze der sekundären $C_{12}$- bis $C_{16}$-Alkansulfonate, das beispielsweise von der Firma Hoechst AG, Frankfurt/Deutschland unter der Handelsbezeichnung Hostapur® SAS 30 in Form einer 30prozentigen Lösung vertrieben wird, bevorzugt. Von den geeigneten Alkylbenzolsulfonaten ist das Natriumsalz des linearen Dodecylbenzolsulfonates, das beispielsweise in Form einer 55prozentigen Paste unter der Handelsbezeichnung Maranil® Paste A 55 von der Firma Henkel KGaA, Düsseldorf/Deutschland vertrieben wird, bevorzugt. Von den geeigneten Xylolsulfonaten ist das Natriumsalz des Xylolsulfonates, welches beispielsweise von der Firma Albright & Wilson Ltd., Cumbria/Großbritannien in Form eines Feststoffes unter der Handelsbezeichnung Eltesol® SX 93 vertrieben wird, bevorzugt. Von den geeigneten Alkylethersulfaten ist jenes bevorzugt, das einen $C_{12}$-Alkylrest aufweist und mit 3 Ethylenoxideinheiten ethoxyliert ist. Ein für die Verwendung als Komponente (b) des erfindungsgemäßen Mittels geeignetes mit 3 Ethylenoxideinheiten ethoxyliertes Laurylethersulfat wird beispielsweise in Form einer 70prozentigen wäßrigen Lösung unter der Handelsbezeichnung Texapon® N 70 von der Firma Henkel KGaA, Düsseldorf/Deutschland vertrieben. Von den geeigneten ethoxylierten Sulfobernsteinsäurehalbestern ist derjenige bevorzugt, der mit drei Ethylenoxideinheiten ethoxyliert ist und einen Alkylrest mit 12 Kohlenstoffatomen aufweist.

Ein mit 3 Ethylenoxideinheiten ethoxylierter Laurylsulfobernsteinsäurehalbester wird beispielsweise in Form einer 40prozentigen wäßrigen Lösung seines Dinatriumsalzes unter der Handeslbezeichnung Texapon® SB3 von der Firma Henkel KGaA, Düsseldorf/Deutschland vertrieben. Von den Alkylethercarboxylaten, die als Komponente (b) in dem erfindungsgemäßen Mittel enthalten sein können, ist jenes bevorzugt, das mit 10 Ethylenoxideinheiten ethoxyliert ist und einen Alkylrest mit 12 Kohlenstoffatomen aufweist. Ein mit 10 Ethylenoxideinheiten ethoxyliertes Laurylethercarboxylat wird in Form einer 22prozentigen wäßrigen Lösung seines Natriumsalzes beispielsweise von der Firma Hüls AG, Marl/Deutschland unter der Handelsbezeichnung Marlinat CM105/80 vertrieben.

Das nichtionische Tensid der Komponente (b) ist bevorzugt ausgewählt aus oxethylierten Fettalkoholen mit 12 bis 18 Kohlenstoffatomen, zum Beispiel mit bis zu 40 Mol Ethylenoxid pro Mol Fettalkohol oxethylierter Lauryl-, Tetradecyl-, Cetyl-, Oleyl- und Stearylalkohol, allein oder im Gemisch; die Fettalkohole von oxethyliertem Lanolin oder oxethyliertes Lanolin: Polyglycerylether von gesättigten oder ungesättigten Fettalkoholen und Alkylphenolen mit 8 bis 30 Kohlenstoffatomen im Alkylrest und 1 bis 10 Glyceryleinheiten im Molekül; Fettsäurealkanolamide und oxethylierte Sorbitanfettsäureester. Besonders geeignet als nichtionische Tenside sind außerdem natürliche Tenside wie Alkylpolyglucoside, die beispielsweise unter der Handelsbezeichnung Plantaren APG 600 von der Firma Henkel/Deutschland vertrieben werden sowie natürliche Tenside wie sie beispielsweise unter der Handelsbezeichnung Oramix NS 10 von der Firma Seppic vertrieben werden.

Als amphotere Tenside der Komponente (b) sind generell Derivate aliphatischer quaternärer Ammonium-, Phosphonium- und Sulfoniumverbindungen der Formel (IV) geeignet,

$$R^2-\overset{\overset{\displaystyle (R^3)_x}{|}}{Y^{(+)}}-CH_2-R^4-Z^{(-)} \qquad (IV)$$

wobei $R^2$ eine geradkettige oder verzweigtkettige Alkyl-, Alkenyl- oder Hydroxyalkylgruppe mit 8 bis 18 Kohlenstoffatomen und 0 bis etwa 10 Ethylenoxideinheiten und 0 bis 1 Glycerineinheit darstellt; Y eine N-, P- oder S-haltige Gruppe ist; $R^3$ eine Alkyl- oder Monohydroxyalkylgruppe mit 1 bis 3 Kohlenstoffatomen ist; X gleich 1 ist, falls Y ein Schwefelatom ist und X gleich 2 ist, wenn Y ein Stickstoffatom oder ein Phosphoratom ist; $R^4$ eine Alkyl- oder Hydroxyalkylgruppe mit 1 bis 4 Kohlenstoffatomen ist und Z eine Carboxylat-, Sulfat-, Phosphonat- oder Phosphatgruppe darstellt.

Andere amphotere Tenside wie Betaine sind ebenso geeignet für das erfindungsgemäße Haarbehandlungsmittel. Beispiele für Betaine umfassen $C_8$- bis $C_{18}$-Alkylbetaine wie Cocodimethylcarboxymethylbetain, Lauryldimethylcarboxrnethylbetain, Lauryldimethylalphacarboxyethylbetain, Cetyldimethylcarboxymethylbetain, Oleyldimethylgammacarboxypropylbetain und Laurylbis-(2-hydroxypropyl)alphacarboxyethylbetain; $C_8$- bis $C_{18}$-Sulfobetaine wie Cocodimethylsulfopropylbetain, Stearyldimethylsulfopropylbetain, Lauryldimethylsulfoethylbetain, Laurylbis-(2-hydroxyethyl)sulfopropylbetain; die Carboxylderivate des Imidazols, die $C_8$- bis $C_{18}$-Alkyldimethylammoniumacetate, die $C_8$- bis $C_{18}$-Alkyldimethylcarbonylmethylammoniumsalze sowie die $C_8$- bis $C_{18}$-Fettsäurealkylamidobetaine wie beispielsweise das Kokosfettsäureamidopropylbetain, welches beispielsweise in Form einer 30prozentigen wäßrigen Lösung unter der Handelsbezeichnung Tego Betain® L7 der Firma Th. Goldschmidt AG/Deutschland vertrieben wird und das N-Kokosfettsäureamidoethyl-N-[2-(carboxymethoxy) ethyl]-glycerin (CTFA-Name: Cocoamphocarboxyglycinate), welches zum Beispiel in Form einer 50prozentigen wäßrigen Lösung unter der Handelsbezeichnung Miranol® C2M von der Firma Miranol Chemical Co. Inc., New Jersey/USA vertrieben wird.

Geeignete kationische Tenside der Komponente (b) enthalten Aminogruppen oder quaternisierte hydrophile Ammoniumgruppen, welche in Lösung eine positive Ladung tragen und durch die generelle Formel (V) dargestellt werden können,

$$\begin{array}{c} R^1 \\ | \\ R^2\text{—}N^{(+)}\text{—}R^3 \\ | \\ R^4 \end{array} \quad X^{(-)} \qquad (V)$$

wobei $R^1$ bis $R^4$ unabhängig voneinander aliphatische Gruppen mit 1 bis 22 Kohlenstoffatomen, oder eine aromatische Gruppe, eine Alkoxygruppe, eine Polyoxyalkylengruppe, eine Alkylamidogruppe, eine Hydroxyalkylgruppe, eine Arylgruppe oder eine Alkarylgruppe mit 12 bis 22 Kohlenstoffatomen ist und X ein Anion darstellt, beispielsweise ein Halogen, Acetat, Phosphat, Nitrat oder Alkylsulfat. Die aliphatischen Gruppen können zusätzlich zu den Kohlenstoffatomen und den Wasserstoffatomen auch Querverbindungen oder andere Gruppen wie beispielsweise weitere Aminogruppen enthalten.

Beispiele für die als Komponente (b) des erfindungsgemäßen Mittels geeignete „kationische" Tenside sind die Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, beispielsweise Lauryl- oder Cetylpyridiniumchlorid, Alkylamidoethyltrimethylammoniumethersulfate sowie Verbindungen mit kationischem Charakter wie Aminoxide, beispielsweise Alkylmethylaminoxide oder Alkylaminoethyldimethylaminoxide. Besonders bevorzugt sind Cetyltrimethylammoniumchlorid, das beispielsweise in Form einer 26prozentigen wäßrigen Lösung unter der Handelsbezeichnung Dehyquart® A von der Firma Henkel KGaA, Düsseldorf/Deutschland und unter der Handelsbezeichnung Genamin® CTAC von der Firma Hoechst AG, Frankfurt/Deutschland sowie in Form einer 50prozentigen Lösung in Isopropanol unter der Handelsbezeichnung Arquad® 16-50 von der Firma Akzo Chemicals GmbH, Düren/Deutschland vertrieben wird.

Eine bevorzugte Ausführungsform des erfindungsgemäßen Haarreinigungsmittels enthält zusätzlich zu den Komponenten (a) und (b) ein Lösungsmittel oder ein Gemisch aus Lösungsmitteln mit einem Siedepunkt unter 400 Grad Celsius in einer Menge von 0,5 bis 90 Gewichtsprozent, bevorzugt von 10 bis 50 Gewichtsprozent. Geeignet sind unverzweigte oder verzweigte Kohlenwasserstoffe mit 5 bis 15 Kohlenstoffatomen wie Pentan, Hexan, Isopentan und zyklische Kohlenwasserstoffe wie Cyclopentan und Cyclohexan, wobei Pentan besonders bevorzugt ist.

Besonders geeignet sind außerdem geradkettige` zyklische oder verzweigtkettige Alkohole wie Ethanol, Isopropanol. Weiterhin geeignet sind hochsiedende Lösungsmittel wie Ethylenglykol oder Propylenglykol. Insbesondere kann das erfindungsgemäße Haarbehandlungsmittel in Aerosolform eingesetzt werden, wobei Treibgase wie Propan-Butan, Dimethylether oder fluorierte Kohlenwasserstoffe als Treibmittel mit teilweisem Lösungsmittelcharakter, in einer Menge von etwa 3 bis 90 Gewichtsprozent, bevorzugt von 5 bis 15 Gewichtsprozent eingesetzt werden können.

Eine weitere bevorzugte Ausführungsform des erfindungsgemäßen Haarreinigungsmittels enthält zusätzlich mindestens ein weiteres Silikonderivat in einer Menge von vorzugsweise 0,1 bis 40 Gew.%. Geeignete Silikonderivate sind beispielsweise lineare Polydimethylsiloxane (CTFA-Bezeichnung: Dimethicone), cyclische Polydimethylsiloxane (CTFA-Bezeichnung: Cyclomethicone); Polydimethylsiloxane mit Hydroxylendgruppen (CTFA-Bezeichnung: Dimethiconole) oder Dimethylsiloxanglykolcopolymere (CTFA-Bezeichnung: Dimethicon Copolyole). Polydimethylsiloxane werden zum Beispiel unter den Handelsnamen Dow Corning Silicone Fluids 200, 225, 244, 245, 344, 345, VS-7207, VS-7349, VS-7158 der Firma Dow Corning/USA, Abil® K4, Abil® 10-10000 der Firma Goldschmidt/Deutschland, GE Silicone SF 1202 oder 1173 der Firma GE Silicones/USA oder Belsil® DMC der Firma Wacker/Deutschland vertrieben. Dimethylsiloxanglykolcopolymere werden zum Beispiel unter den Handelsnamen Abil® -Polyether-Polysiloxan der Firma Goldschmidt/Deutschland, Silwet-L der Firma Union Carbide/USA oder Silikonfluids 190, 193, Q2-5520 der Firma Dow Corning/USA vertrieben. Weiterhin können Cetyl Dimethicon oder Cetyl Dimethicon Copolyol enthalten sein.

Besonders bevorzugte Silikonderivate sind die Polydimethylsiloxane mit Hydroxylendgruppen (Dimethiconole). Geeignete Dimethiconole werden zum Beispiel unter den Handelsnamen Silicone Fluid F 212 oder Siliconöl CT 601 M der Firma Wacker/Deutschland, Silicone Fluid Serie NM 201-50000 der Firma Hüls/Deutschland, die S-Serien der

Firma Siltech/USA, Unisil SF-R der Firma UPI/USA vertrieben. Geeignete Gemische mit leichtflüchtigen Cyclomethiconen oder Dimethiconen sind die Handelsprodukte Dow Corning Q2-1401 und Dow Corning Q2-1403 der Firma Dow Corning/USA und Abil® OSW 12 und 13 der Firma Goldschmidt/Deutschland. Durch den Gehalt an Dimethiconolen werden die festigenden Eigenschaften des erfindungsgemäßen Haarreinigungsmittels gesteigert und das Rückstandsverhalten auf dem Haar wird positiv beeinflußt.

Zusätzlich zu den Komponenten (a) und (b) kann das erfindungsgemäße Haarreinigungsmittel Polymere mit stark verdickender Wirkung in einer Menge von 0,01 bis 20 Gewichtsprozent enthalten. Beispiele für geeignete Polymere mit stark verdickender Wirkung sind Homopolymere der Acrylsäure mit einem Molekulargewicht von 2.000.000 bis 6.000.000, die beispielsweise von der Firma BF Goodrich, Cleveland/USA unter der Handelsbezeichnung Carbopol® vertrieben werden. Weitere Beispiele sind das unter dem Handelsnamen Hostacerin® PN 73 vertriebene Polymer aus Acrylsäure und Acrylamid (Natriumsalz) von der Firma Hoechst/Deutschland mit einem Molekulargewicht von 2000.000 bis 6.000.000, Guar Gum, 2-Hydroxypropylether substituiertes Guar Gum (CTFA-Bezeichnung: Hydroxypropyl Guar), Karaya Gum, Xanthan Gum und das von der Firma Alban Muller, Montreuil/Frankreich unter dem Handelsnamen Amigel® vertriebene Sclerotium Gum. Weitere Verdicker sind die Copolymere der Acrylsäure oder der Methacrylsäure wie sie beispielsweise unter den Handelsnamen Carbopol 1342 oder Pemulen TR1 von der Firma Goodrich/USA vertrieben werden. Besonders bevorzugt enthält das erfindungsgemäße Mittel 2-Hydroxy-3-(trimethylammoniumchlorid)propylether substituiertes Guar Gum (CTFA-Bezeichnung: Guar Hydroxypropyltrimonium Chloride) oder verdickende Celluloseether wie beispielsweise Hydroxypropylcellulose, Hydroxyethyl cellulose, in einer Menge von etwa 0,05 bis 5 Gewichtsprozent, wie sie zum Beispiel unter dem Handelsnamen Natrosol 250 HHR von der Firma Aqualon vertrieben wird.

Zusätzlich zu den Komponenten (a) und (b) kann das erfindungsgemäße Haarreinigungsmittel anionische, kationische, amphotere oder nichtionische filmbildende Polymere, in einer Menge von 0,01 bis 20 Gewichtsprozent enthalten, die in der Formulierung gelöst, teilweise gefällt oder ungelöst vorliegen und filmbildende oder verdickende oder viskositätserniedrigende Wirkung aufweisen, wobei die anionischen oder kationischen und amphoteren Polymere unneutralisiert, teilweise neutralisiert oder voll neutralisiert vorliegen können. Dabei können alle synthetisch hergestellten, natürlichen oder chemisch modifizierten natürlichen Polymere verwendet werden, die mit den im erfindungsgemäßen Haarbehandlungsmittel aufgeführten Inhaltsstoffen verträglich sind.

Beispiele für geeignete nichtionische Polymere sind Polyvinylpyrrolidon-Polymere, die beispielsweise von der Firma BASF AG, Ludwigshafen/Deutschland unter der Handelsbezeichnung Luviskol® vertrieben werden; Copolymerisate aus Vinylpyrrolidon und Vinylacetat, die beispielsweise unter den Handelsbezeichnungen Luviskol® VA von der Firma BASF, Ludwigshafen/Deutschland vertrieben werden; Terpolymere aus Vinylpyrrolidon, Vinylacetat und Vinylpropionat, die beispielsweise unter der Handelsbezeichnung Luviskol® VAP der Firma BASF, Ludwigshafen/Deutschland vertrieben werden; Polyacrylamide, die beispielsweise unter den Handelsbezeichnungen Akypomine® P191 von der Firma CHEM-Y, Emmerich/Deutschland oder Sepigel® 305 von der Firma Seppic/USA vertrieben werden; Polyvinylalkohole, die beispielsweise unter den Handelsbezeichnungen Elvanol® der Firma Du Pont oder Vinol® 523/540 der Firma Air Products/USA vertrieben werden sowie Polyethylenglykole mit einem Molekulargewicht von 800 bis 20.000 g/mol, die beispielsweise unter den Handelsbezeichnungen Lipoxol® 1000 von der Firma Hüls AG/Deutschland, Pluracol E 4000 von der Firma BASF/Deutschland oder Upiwax® 20.000 von der Firma UPI vertrieben werden. Weiterhin sind die Homopolymere des N-Vinylformamids, wie sie beispielsweise unter den Handelsbezeichnungen PVF von der Firma National Starch/USA vertrieben werden geeignet. Als ein nichtionisches Polymer natürlichen Ursprungs ist beispielsweise Hydroxypropylchitosan zu nennen.

Beispiele für geeignete anionische Polymere sind Crotonsäure-Vinylacetat-Copolymere, die beispielsweise in Form einer 60prozentigen Lösung in Isopropanol/Wasser unter der Handelsbezeichnung Aristoflex® von der Firma Hoechst/Deutschland vertrieben werden. Weitere geeignete anionische Polymere sind beispielsweise Terpolymere der Acrylsäure, Ethylacrylat und N-t-Butylacrylamid wie sie unter dem Handelsnamen Ultrahold 8 und Ultrahold Strong der Firma BASF, Ludwigshafen/Deutschland vertrieben werden.

Beispiele für geeignete kationische Polymere sind Vinylpyrrolidon/Methacrylamidopropyltrimethylammoniumchlorid-Copolymere wie sie beispielsweise unter der Handelsbezeichnung Gafquat® von der Firma Gaf Co., New York/USA vertrieben werden. Weitere kationische Polymere sind beispielsweise das von der Firma BASF AG, Ludwigshafen/Deutschland unter dem Handelsnamen Luviquat® HM 550 vertriebene Copolymer aus Polyvinylpyrrolidon und Imidazoliminmethochlorid, das von der Firma Calgon, Pittsburgh/USA unter dem Handelsnamen Merquat® Plus 3330 vertriebene Terpolymer aus Dimethyldiallylammoniumchlorid, Natriumacrylat und Acrylamid, das von der Firma ISP/USA unter dem Handelsnamen Gaffix® VS 713 vertriebene Terpolymer aus Vinylpyrrolidon, Dimethylaminoethylmethacrylat und Vinylcaprolactam, das von der Firma Amerchol, Edison/USA unter dem Handelsnamen Polymer JR® vertriebene quarternisierte Ammoniumsalz der Hydroxyethylcellulose und einem trimethylammonium-substituierten Epoxid, das von der Firma Gaf unter dem Handelsnamen Gafquat® HS 100 vertriebene Vinylpyrrolidon/Methacrylamidopropyltrimethylammoniumchlorid-copolymer und das von der Firma Goldschmidt, Essen/Deutschland unter dem Handelsnamen Abil® Quat 3272 vertriebene diquaternäre Polydimethylsiloxan.

Beispiele für geeignete amphotere Polymere sind Copolymere aus Octylacrylamid, t-Butylaminoethylmethacrylat und einer oder mehreren Monomeren, ausgewählt aus Acrylsäure, Methacrylsäure oder deren Ester wie sie beispielsweise unter dem Handelsnamen Amphomer ® 28-4910 oder Amphomer LV-71 der Firma National Starch/Großbritannien erhältlich sind. Beispiele geeigneter natürlicher Polymere, welche das erfindungsgemäße Haarmittel zusätzlich in einer Menge von 0,1 bis 15 Gewichtsprozent beinhalten kann, sind die niedermolekularen sowie die hochmolekularen Chitosane, welche beispielsweise von der Firma Kyowa Oil & Fat/Japan vertrieben werden. Des weiteren können verschiedene Saccharidtypen verwendet werden wie zum Beispiel Polysaccharide oder Gemische aus Oligo-, Mono- und Disacchariden, welche beispielsweise unter dem Handelsnamen C-PUR® der Firma Cerestar, Brüssel/Belgien vertrieben werden.

Selbstverständlich kann das erfindungsgemäße Haarreinigungsmittel die für die Haarreinigungsmittel oder Stylingmittel üblichen Zusatzstoffe enthalten: Trübungsmittel wie zum Beispiel Ethylenglykoldistearat, in einer Menge von etwa 0,5 bis 5,0 Gewichtsprozent; Perglanzmittel wie zum Beispiel ein Gemisch aus Fettsäuremonoalkylolamid/Ethylenglykoldistearat, in einer Menge von etwa 1,0 bis 10,0 Gewichtsprozent; konservierende Wirkstoffe wie zum Beispiel 2,2,4-Trichlor-2-hydroxy-diphenylether, Methylchloroisothiazolinon, p-Hydroxybenzoesäureester, Sorbinsäure, Salicylsäure, Mandelsäure oder Ameisensäure, in einer Menge von etwa 0,01 bis 1,0 Gewichtsprozent; Verdickungsmittel wie beispielsweise Glycerinmonolaurat, Kokosfettsäurediethanolamid, Natriumchlorid, in einer Menge von etwa 0,5 bis 3,0 Gewichtsprozent; Verdünnungsmittel, wie zum Beispiel 1,2-Propylenglykol oder ethoxyliertes Sorbitanmonolaurat, in einer Menge von etwa 0,5 bis 5,0 Gewichtsprozent; Lichtschutzmittel, Glanzgeber; Puffersubstanzen, wie beispielsweise Natriumcitrat oder Natriumphosphat, in einer Menge von etwa 0,1 bis 1,0 Gewichtsprozent; Lösungsvermittler, wie zum Beispiel ethoxyliertes, gegebenenfalls hydriertes Rizinusöl, in einer Menge von etwa 0,1 bis 1,0 Gewichtsprozent; Antioxidantien wie Tocopherole in einer Menge von etwa 0,01 bis 1 Gewichtsprozent sowie Anfärbestoffe, wie zum Beispiel Fluorescein-Natriumsalz, in einer Menge von etwa 0,1 bis 1,0 Gewichtsprozent. Zusätzlich kann das erfindungsgemäße Haarreinigungsmittel pflegende, feuchtigkeitsspendende Mittel wie 1,2-Propandiol und Glycerin oder konditionierende Zusätze enthalten. Beispiele für konditionierende Zusätze beinhalten insbesondere Verbindungen, welche eine ausgeprägte Substantivität zum Haar aufweisen, wie quaternäre Ammoniumsalze des Guars, Amodimethicone, Dimethicone Copolyol Phosphate, Dimethicone/Disodium PG-Propyldimethicone Thiosulfate Copolyol (zum Beispiel Abil® S 255 der Firma Goldschmidt AG/Deutschland), Dimethicone Propyl PG-Betaine (zum Beispiel Abil® B 9950 der Firma Th. Goldschmidt AG/ Deutschland), in einer Menge von etwa 0,01 bis 10 Gewichtsprozent. Beispiele für pflegende Zusätze sind insbesondere Öle, Wachse oder Fettsäuren, welche aus tierischen oder pflanzlichen Rohstoffen gewonnen werden wie zum Beispiel Jojobaöl oder Fruchtwachse.

Der Wassergehalt des erfindungsgemäßen Haarreinigungsmittels liegt vorzugsweise zwischen 15 und 80 Gewichtsprozent.

Das erfindungsgemäße Mittel weist vorzugsweise einen pH-Wert von 4 bis 9, besonders bevorzugt von 4,5 bis 7,5 auf. Die Einstellung des pH-Wertes des erfindungsgemäßen Mittels kann beispielsweise mit Zitronensäure, Phosphorsäure, Salzsäure oder Natriumhydroxid erfolgen.

Die mit dem erfindungsgemäßen Mittel gereinigten Haare weisen eine gute Naß- und Trockenkämmbarkeit, eine gute Frisierbarkeit sowie eine spürbare Festigung auf, ohne das Haar zu belasten oder den natürlichen Griff des Haares zu beeinträchtigen.

Die folgenden Beispiele sollen den Gegenstand der Erfindung näher erläutern.

**Beispiel 1: Haarreinigungsmittel mit anionischem Tensid**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 8,4 g | Laurylethersulfat |
| 1,0 g | Hydroxyethylcellulose (Natrosol® 250 HHR der Firma Aqualon/Niederlande) |
| 20,0 g | Isopropanol |
| 60,6 g | Wasser |
| 100,0 g | |

**Beispiel 2: Haarreinigungsmittel mit anionischem und amphoterem Tensid**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 7,0 g | Laurylethersulfat |
| 1,5 g | Kokosfettsäureamidopropylbetain |
| 1,0 g | 2-Hydroxy-3-(trimethylammoniumchlorid)-propylether substituiertes Guar Gum |
| 20,0 g | Isopropanol |
| 60,5 g | Wasser |
| 100,0 g | |

**Beispiel 3: Haarreinigungsmittel mit amphoterem Tensid**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 9,0 g | Kokosfettsäureamidopropylbetain |
| 1,0 g | 2-Hydroxy-3-(trimethylammoniumchlorid)-propylether substituiertes Guar Gum |
| 20,0 g | Isopropanol |
| 60,0 g | Wasser |
| 100,0 g | |

**Beispiel 4: Haarreinigungsmittel mit anionischem und nichtionogenem Tensid**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 7,0 g | Laurylethersulfat |
| 2,75 g | Decylglucosid |
| 1,0 g | Hydroxyethylcellulose (Natrosol® 250 HHR der Firma Aqualon/Niederlande) |
| 0,5 g | Polydimethylsiloxan (Silicone Fluids 225® der Firma Dow Corning/USA) |
| 0,5 g | Dimethicon Copolyol (Silwet-Copolymer L 7604 der Firma Union Carbide/USA) |
| 20,0 g | Isopropanol |
| 58,25 g | Wasser |
| 100,0 g | |

**Beispiel 5: Haarreinigungsmittel mit amphoterem und nichtionogenem Tensid**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 10,0 g | Laurylglucosid |
| 6,0 g | Kokosfettsäureamidopropylbetain |
| 1,0 g | 2-Hydroxy-3-(trimethylammoniumchlorid)-propylether substituiertes Guar Gum |
| 20,0 g | Isopropanol |
| 52,0 g | Wasser |

**Beispiel 6: Haarreinigungsmittel mit anionischem Tensid in Aerosolform**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 8,4 g | Laurylethersulfat |
| 1,0 g | Hydroxyethylcellulose (Natrosol® 250 HHR der Firma Aqualon/Niederlande) |
| 0,5 g | Dimethiconol, 13%ig in Cyclomethicon (Abil® OSW 13 der Firma Goldschmidt/Deutschland) |
| 30,0 g | Isopropanol |
| 10,0 g | Propan-Butan Treibgasgemisch |
| 40,1 g | Wasser |
| 100,0 g | |

**Beispiel 7: Haarreinigungsmittel mit anionischem Tensid und amphoterem Tensid in Aerosolform**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 5,6 g | Laurylethersulfat |
| 3,0 g | Kokosfettsäureamidopropylbetain |
| 1,0 g | 2-Hydroxy-3-(trimethylammoniumchlorid)-propylether substituiertes Guar Gum |
| 30,0 g | Isopropanol |
| 10,0 g | Propan-Butan Treibgasgemisch |
| 40,2 g | Wasser |

**Beispiel 8: Haarreinigungsmittel mit anionischem und nichtionischem Tensid in Aerosolform**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 5,6 g | Laurylethersulfat |
| 5,5 g | Decylglucosid |
| 1,0 g | Hydroxyethylcellulose (Natrosol® 250 HHR der Firma Aqualon/Niederlande) |
| 30,0 g | Isopropanol |
| 10,5 g | Propan-Butan Treibgasgemisch |
| 27,4 g | Wasser |

**Beispiel 9: Haarreinigungsmittel mit Pentan-Zusatz**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 8,4 g | Laurylethersulfat |
| 1,0 g | Hydroxyethylcellulose (Natrosol® 250 HHR der Firma Aqualon/Niederlande) |
| 1,0 g | Laurylmethicon Copolyol (Dow Corning® Q2-5200 der Firma Dow Corning, Europe/Belgien) |
| 20,0 g | Isopropanol |
| 20,0 g | Pentan |
| 39,6 g | Wasser |
| 100,0 g | |

**Beispiel 10: Haarreinigungsmittel mit Pentan-Zusatz in Aerosolform**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 8,4 g | Laurylethersulfat |
| 1,0 g | Hydroxyethylcellulose (Natrosol® 250 HHR der Firma Aqualon/Niederlande) |
| 0,5 g | Dimethiconol, 13%ig in Cyclomethicon (Dow Corning® 1401 der Firma Dow Corning, Europe/Belgien) |
| 0,5 g | Laurylmethicon Copolyol (Dow Corning® Q2-5200 der Firma Dow Corning, Europe/Belgien) |
| 20,0 g | Isopropanol |
| 20,0 g | Pentan |
| 39,6 g | Wasser |

(fortgesetzt)

| | |
|---|---|
| 100,0 g | |

**Beispiel 11: Haarreinigungsmittel in 2-Komponenten-Form**

Komponente 1:

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 2,0 g | Acrylat/$C_{10}$-$_{30}$-Alkylacrylat Crosspolymer (Pemulen® TR-1 der Firma Goodrich/USA) |
| 1,0 g | Dimethiconol, 13%ig in Cyclomethicon (Dow Corning® 1401 der Firma Dow Corning, Europe/Belgien) |
| 40,0 g | Isopropanol |
| 47,0 g | Wasser |
| 100,0 g | |

Komponente 2: Laurylethersulfat, 28%ig in Wasser

Wird Komponente 1 zusammen mit Komponente 2 auf das Haar aufgebracht, erfolgt eine Wirkung gemäß oben dargestellten erfindungsgemäßen Vorteilen des vorliegenden Haarbehandlungsmittels.

**Beispiel 12: Haarreinigungsmittel mit anionischem Tensid**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 8,4 g | Laurylethersulfat |
| 1,0 g | Dimethiconol, 13%ig in Cyclomethicon (Dow Corning® 1401 der Firma Dow Corning, Europe/Belgien) |
| 1,0 g | Hydroxyethylcellulose (Natrosol® 250 HHR der Firma Aqualon/Niederlande) |
| 20,0 g | Isopropanol |
| 59,6 g | Wasser |
| 100,0 g | |

**Beispiel 13: Haarreinigungsmittel mit anionischem und amphoterem Tensid**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 7,0 g | Laurylethersulfat |

(fortgesetzt)

| | |
|---|---|
| 1,5 g | Kokosfettsäureamidopropylbetain |
| 1,0 g | Dimethiconol, 13%ig in Cyclomethicon (Dow Corning® 1401 der Firma Dow Corning, Europe/Belgien) |
| 1,0 g | 2-Hydroxy-3-(trimethylammoniumchlorid)-propylether substituiertes Guar Gum |
| 20,0 g | Isopropanol |
| 59,5 g | Wasser |
| 100,0 g | |

**Beispiel 14: Haarreinigungsmittel mit amphoterem Tensid**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 9,0 g | Kokosfettsäureamidopropylbetain |
| 1,0 g | Dimethylsiloxanglykolcopolymer (Silwet® Copolymer PC 90 der Firma Union Carbide/USA) |
| 1,0 g | 2-Hydroxy-3-(trimethylammoniumchlorid)-propylether substituiertes Guar Gum |
| 20,0 g | Isopropanol |
| 59,0 g | Wasser |
| 100,0 g | |

**Beispiel 15: Haarreinigungsmittel mit amphoterem und nichtionogenem Tensid**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 10,0 g | Laurylglucosid |
| 6,0 g | Kokosfettsäureamidopropylbetain |
| 1,0 g | Dimethiconol, 13%ig in Cyclomethicon (Dow Corning® 1401 der Firma Dow Corning, Europe/Belgien) |
| 1,0 g | 2-Hydroxy-3-(trimethylammoniumchlorid)-propylether substituiertes Guar Gum |
| 20,0 g | Isopropanol |
| 51,0 g | Wasser |

**Beispiel 16: Haarreinigungsmittel mit anionischem Tensid und amphoterem Tensid in Aerosolform**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |

(fortgesetzt)

| | |
|---|---|
| 5,6 g | Laurylethersulfat |
| 3,0 g | Kokosfettsäureamidopropylbetain |
| 1,0 g | 2-Hydroxy-3-(trimethylammoniumchlorid)-propylether substituiertes Guar Gum |
| 0,5 g | Dimethiconol, 13%ig in Cyclomethicon (Abil® OSW 13 der Firma Goldschmidt/Deutschland) |
| 30,0 g | Isopropanol |
| 10,0 g | Propan-Butan Treibgasgemisch |
| 39,7 g | Wasser |

**Beispiel 17: Haarreinigungsmittel mit anionischem und nichtionischem Tensid in Aerosolform**

| | |
|---|---|
| 10,0 g | Dimethylsiloxan/Methyl-3-mercaptopropylsiloxan/Isobutylmethacrylat Copolymer, 25%ig in Cyclomethicon (Polymer Plus VS70® der Firma 3M Corp./USA) |
| 5,6 g | Laurylethersulfat |
| 5,5 g | Decylglucosid |
| 1,0 g | Hydroxyethylcellulose (Natrosol® 250 HHR der Firma Aqualon/Niederlande) |
| 0,5 g | Dimethylpolysiloxan (Viskosität 500 mPa • s bei 25 °C) |
| 30,0 g | Isopropanol |
| 10,5 g | Propan-Butan Treibgasgemisch |
| 26,9 g | Wasser |

Soweit nichts anderes vermerkt ist, bedeuten alle Prozentangaben Gewichtsprozent.

**Patentansprüche**

**1.** Haarreinigungsmittel mit einem Gehalt an

(a) mindestens einem nichtionischen, wasserunlöslichen Vinyl/Silikon Copolymer mit einem Grundgerüst aus polymeren Siloxaneinheiten und Seitenketten aus polymeren Vinyleinheiten und

(b) mindestens einem waschaktiven Tensid, ausgewählt aus der Gruppe bestehend aus nichtionischen, anionischen, kationischen und amphoteren Tensiden.

**2.** Mittel nach Anspruch 1, dadurch gekennzeichnet, daß das Vinyl/Silikon Copolymer die allgemeine Formel (I)

$$R^1R^2R^3Si\text{-}[O\text{-}SiR^4R^5\text{-}]_nO\text{-}SiR^6R^7R^8 \quad (I),$$

aufweist, wobei die Reste $R^1$ bis $R^8$ gleich oder verschieden sein können und unabhängig voneinander für eine Alkyl-, Aryl-, Alkylaryl-, Alkoxy-, Alkylamino-, Fluoroalkyl-, Wasserstoff-, Hydroxy- oder für eine ZSA-Gruppe stehen, Z für eine divalente Verbindungsgruppe, S für Schwefel und A für ein aus nichtionischen Monomeren aufgebautes Vinylpolymer-Segment steht und n eine Zahl größer oder gleich 5 bedeutet mit der Maßgabe, daß mindestens einer der Reste $R^1$ bis $R^8$ für eine ZSA-Gruppe steht.

**3.** Mittel nach Anspruch 2, dadurch gekennzeichnet, daß die nichtionischen Monomeren des Vinylpolymer-Segments A ausgewählt sind aus niedrigpolaren Estern der Acrylsäure oder der Methacrylsäure mit Alkoholen kurzer bis mittlerer Kettenlänge.

4. Mittel nach Anspruch 3, dadurch gekennzeichnet, daß das Vinyl/Silikon Copolymer ein Dimethylsiloxan/Methyl-3-mercaptopropylsiloxan/Isobutylmethacrylat Copolymer ist.

5. Mittel nach einem der Ansprüche 1 bis 4, dadurch gekennzeichnet, daß das Vinyl/Silikon Copolymer (a) in einer Konzentration von 0,1 bis 30 Gewichtsprozent und das Tensid (b) in einer Konzentration von 3 bis 50 Gewichtsprozent vorliegt.

6. Mittel nach einem der Ansprüche 1 bis 5, dadurch gekennzeichnet, daß es zusätzlich mindestens ein Silikonderivat enthält.

7. Mittel nach Anspruch 6, dadurch gekennzeichnet, daß das Silikonderivat ausgewählt ist aus Polydimethylsiloxanen, Polydimethylsiloxanen mit Hydroxylendgruppen und Dimethylsiloxanglykolcopolymeren.

8. Mittel nach Anspruch 7, dadurch gekennzeichnet, daß das Silikonderivat ein Polydimethylsiloxan mit Hydroxylendgruppen ist.

9. Mittel nach einem der Ansprüche 6 bis 8, dadurch gekennzeichnet, daß das Silikonderivat in einer Menge von 0,1 bis 40 Gewichtsprozent enthalten ist.

10. Mittel nach einem der Ansprüche 1 bis 9, dadurch gekennzeichnet, daß zusätzlich mindestens ein unverzweigter oder verzweigter Kohlenwasserstoff mit 5 bis 15 Kohlenwasserstoffatomen enthalten ist.

## Claims

1. Hair cleaning agent containing

(a) at least one nonionic, water-insoluble vinyl/silicone copolymer having a backbone of polymeric siloxane units and side chains of polymeric vinyl units and

(b) at least one detergent surfactant, selected from the group consisting of nonionic, anionic, cationic and amphoteric surfactants.

2. Agent according to claim 1, characterised in that the vinyl/silicone copolymer has the general formula (I)

$$R^1R^2R^3Si\text{-}[O\text{-}SiR^4R^5\text{-}]_nO\text{-}SiR^6R^7R^8 \quad (I),$$

wherein the residues $R^1$ to $R^8$ may be identical or different and, independently of one another, represent an alkyl, aryl, alkylaryl, alkoxy, alkylamino, fluoroalkyl, hydrogen, hydroxy or ZSA group, Z represents a divalent compound group, S represents sulphur and A represents a vinyl polymer segment synthesised from nonionic monomers and n represents a number greater than or equal to 5 with the proviso that at least one of the residues $R^1$ to $R^8$ represents a ZSA group.

3. Agent according to claim 2, characterised in that the nonionic monomers of the vinyl polymer segment A are selected from low-polarity esters of acrylic acid or methacrylic acid with alcohols of short to medium chain length.

4. Agent according to claim 3, characterised in that the vinyl/silicone copolymer is a dimethylsiloxane/methyl-3-mercaptopropylsiloxane/isobutyl methacrylate copolymer.

5. Agent according to any one of claims 1 to 4, characterised in that the vinyl/silicone copolymer (a) is present in a concentration of from 0.1 to 30% by weight and the surfactant (b) is present in a concentration of from 3 to 50% by weight.

6. Agent according to any one of claims 1 to 5, characterised in that it additionally contains at least one silicone derivative.

7. Agent according to claim 6, characterised in that the silicone derivative is selected from polydimethylsiloxanes, polydimethylsiloxanes having hydroxyl end groups and dimethylsiloxane glycol copolymers.

8. Agent according to claim 7, characterised in that the silicone derivative is a polydimethylsiloxane having hydroxyl end groups.

9. Agent according to any one of claims 6 to 8, characterised in that the silicone derivative is present in an amount of from 0.1 to 40% by weight.

10. Agent according to any one of claims 1 to 9, characterised in that, additionally, at least one unbranched or branched hydrocarbon having from 5 to 15 hydrocarbon atoms is present.

## Revendications

1. Produit pour le nettoyage des cheveux, contenant :

   (a) au moins un copolymère vinylique/silicone nonionique, insoluble dans l'eau, ayant un squelette constitué de motifs siloxane polymères et de chaînes latérales constituées de motifs vinyliques polymères, et
   (b) au moins un tensioactif détergent, choisi dans l'ensemble comprenant les tensioactifs nonioniques, anioniques, cationiques et amphotères.

2. Produit selon la revendication 1, caractérisé en ce que le copolymère vinylique/silicone a la formule générale (I)

$$R^1R^2R^3Si\text{-}[O\text{-}SiR^4R^5\text{-}]_nO\text{-}SiR^6R^7R^8 \quad (I)$$

dans laquelle les radicaux $R^1$ à $R^8$ peuvent être identiques ou différents et représentent chacun, indépendamment des autres, un groupe alkyle, aryle, alkylaryle, alcoxy, alkylamino, fluoralkyle, ou un atome d'hydrogène ou un groupe hydroxy ou ZSA, où Z est un groupe de liaison divalent, S est le soufre et A représente un segment d'un polymère vinylique constitué de monomères non-ioniques, et n est un nombre supérieur ou égal à 5, à la condition qu'au moins l'un des radicaux $R^1$ à $R^8$ soit un groupe ZSA.

3. Produit selon la revendication 2, caractérisé en ce que les monomères non-ioniques du segment polymère vinylique A sont choisis parmi les esters à faible polarité de l'acide acrylique ou de l'acide méthacrylique et d'alcools à longueur chaîne courte à moyenne.

4. Produit selon la revendication 3, caractérisé en ce que le copolymère vinylique/silicone est un copolymère diméthylsiloxane/méthyl-3-mercaptopropylsiloxane/méthacrylate d'isobutyle.

5. Produit selon l'une des revendications 1 à 4, caractérisé en ce que le copolymère vinylique/silicone (a) est présent à une concentration de 0,1 à 30 % en poids, et le tensioactif (b) est présent à une concentration de 3 à 50 % en poids.

6. Produit selon l'une des revendications 1 à 5, caractérisé en ce qu'il contient en outre au moins un dérivé de silicone.

7. Produit selon la revendication 6, caractérisé en ce que le dérivé de silicone est choisi parmi les polydiméthylsiloxanes, les polydiméthylsiloxanes comportant des groupes terminaux hydroxyle, et les copolymères diméthylsiloxane-glycols.

8. Produit selon la revendication 7, caractérisé en ce que le dérivé de silicone est un polydiméthylsiloxane ayant des groupes terminaux hydroxyle.

9. Produit selon l'une des revendications 6 à 8, caractérisé en ce que le dérivé de silicone est présent en une quantité de 0,1 à 40 % en poids.

10. Produit selon l'une des revendications 1 à 9, caractérisé en ce qu'il contient en outre au moins un hydrocarbure à chaîne droite ou ramifiée ayant de 5 à 15 atomes de carbone.